# EUROPÄISCHE PATENTSCHRIFT

(11) **EP 1 581 259 B1**
(45) Veröffentlichungstag und Bekanntmachung des Hinweises auf die Patenterteilung: **18.08.2010**
(21) Anmeldenummer: 02795051.8
(22) Anmeldetag: 28.12.2002
(51) Int. Cl.: A61K 47/36, A61K 47/02, A61K 31/00, A61K 38/00, A23L 1/05, A61K 9/14, A61P 29/00, A61P 31/12, A61P 1/00

(54) **VERFAHREN SOWOHL ALS STOFF ZUR SPEZIFISCHEN PROLIFERATIONSFÖRDERUNG VON CD4+ LYMPHOZYTEN DURCH EINE KOMBINATION VON ALGINAT MIT AN HOCHDISPERSE KIESELSAEURE GEKOPPELTEM CURCUMIN**
METHOD AND MATERIAL FOR THE SPECIFIC INDUCTION OF PROLIFERATION OF CD4+ LYMPHOCYTES USING A COMBINATION OF ALGINIC ACID WITH COLLOIDAL SILICA-BOUND CURCUMIN
MÉTHODE ET MATÉRIEL POUR L'INDUCTION SPÉCIFIQUE DE LA PROLIFÉRATION DES LYMPHOCYTES CD4+ UTILISANT UNE COMBINAISON D'ACIDE ALGINIQUE ET DE CURCUMINE ATTACHEE A DE LA SILICE COLLOïDALE

(43) Veröffentlichungstag der Anmeldung: 05.10.2005
(73) Patentinhaber: Gradl-Grams, Marianne, 94371 Rattenberg (DE)
(72) Erfinder: GRADL, Toni, 93437 Furth im Wald (DE)
(74) Vertreter: advotec.
(86) Internationale Anmeldenummer: PCT/DE2002/004751
(87) Internationale Veröffentlichungsnummer: WO 2004/060402

(56) Entgegenhaltungen:
- WO-A1-01/14468
- DE-A- 4 435 525
- DE-A- 19 723 155
- KANG D K ET AL: "Peroral immunization of microencapsulated human VP8 in combination with cholera toxin induces intestinal antibody responses." MOLECULES AND CELLS. KOREA (SOUTH) 31 DEC 1999, Bd. 9, Nr. 6, 31. Dezember 1999 (1999-12-31), Seiten 609-616, XP009019833 ISSN: 1016-8478
- CHURCHILL M ET AL: "Inhibition of intestinal tumors by curcumin is associated with changes in the intestinal immune cell profile." THE JOURNAL OF SURGICAL RESEARCH. UNITED STATES APR 2000, Bd. 89, Nr. 2, April 2000 (2000-04), Seiten 169-175, XP002256270 ISSN: 0022-4804
- DATABASE WPI Section Ch, Week 199042 Derwent Publications Ltd., London, GB; Class A14, AN 1990-317948 XP002258923 & JP 02 228362 A (HIGASHI NIHON BUSSA), 11. September 1990 (1990-09-11)
- VLIETNICK A J ET AL: "Plant-derived leading compounds for chemotherapy of human immunodeficiency virus (HIV) infection." PLANTA MEDICA, Bd. 64, Nr. 2, März 1998 (1998-03), Seiten 97-109, XP001028147 ISSN: 0032-0943
- ALLEN PATRICIA C ET AL: "Dietary modulation of avian coccidiosis." INTERNATIONAL JOURNAL FOR PARASITOLOGY, Bd. 28, Nr. 7, Juli 1998 (1998-07), Seiten 1131-1140, XP002256271 ISSN: 0020-7519
- BRATTIG N W ET AL: "Immunoenhancing effect of flavonoid compounds on lymphocyte proliferation and immunoglobulin synthesis." INTERNATIONAL JOURNAL OF IMMUNOPHARMACOLOGY. ENGLAND 1984, Bd. 6, Nr. 3, 1984, Seiten 205-215, XP009019476 ISSN: 0192-0561
- BOUIC P J D ET AL: "Beta-sitosterol and beta-sitosterol glucoside stimulate human peripheral blood lymphocyte proliferation: Implications for their use as an immunomodulatory vitamin combination" INTERNATIONAL JOURNAL OF IMMUNOPHARMACOLOGY, Bd. 18, Nr. 12, 1996, Seiten 693-700, XP001155721 ISSN: 0192-0561
- YU RINA ET AL: "Modulation of select immune responses by dietary capsaicin" INTERNATIONAL JOURNAL FOR VITAMIN AND NUTRITION RESEARCH, Bd. 68, Nr. 2, 1998, Seiten 114-119, XP009019474 ISSN: 0300-9831
- VARGA ZOLTAN ET AL: "The effect of juglone on the membrane potential and whole-cell K+ currents of human lymphocytes" BIOCHEMICAL AND BIOPHYSICAL RESEARCH COMMUNICATIONS, Bd. 218, Nr. 3, 1996, Seiten 828-832, XP002258971 ISSN: 0006-291X
- FIMOGNARI C. ET AL: "Cyclin D3 and p53 mediate suforaphane-induced cell cyle delay and apoptosis in non-transformed human T-lymphocytes" CELL MOL LIFE SCI, vol. 59, no. 11, November 2002 (2002-11), pages 2004-2012,
- GRADL T. ET AL: "MENGEN- UND SPURENELEMENTE. MENGEN-UND SPURENELEMENTE. 20. ARBEITSTAGUNG, 1-2.12.2000, FRIEDERICH SCHILLER UNIVERSITÄT JENA" 2 December 2000 (2000-12-02), MANFRED ANKE , MACRO AND TRACE ELEMENTS AS CENTRAL CATIONS IN PHASE TRANSFER CATALYSTS AND THEIR BIOLOGICAL MEANING , XP001536477 * pages 150-153 *
- GRADL T.; BALLAN K.; MAURER S.: "Vitamine und Zusatzstoffe in der Ernährung von Mensch und Tier. 7. Symposium 22. und 23. September 1999 Jena /Thüringen; Seiten 256-261" December 1999 (1999-12), FRANK, DR. (DEZ. 1999) , IMMUNOMODULATION DURCH FUTTERZUSATZSTOFFE BEI LANDWIRTSCHAFTLICHEN NUTZTIEREN , XP009085831 * the whole document *
- NIRMALA C. ET AL: "Cucumin treatment modulates collagen metabolism in isopreterenol induced myocardial necrosis in rats" MOLECULAR AND CELLULAR BIOCHEMISTRY, vol. 197, 1999, page 31-37,
- BINA JOE: "Role of capsaicin, curcumin and dietary n-3 fatty acids in lowering the generation of reactive oxygen species in rat peritoneal macrophages" BIOCHIMICA ET BIOPHYSICA ACTA, vol. 1224, 1994, page 255-263,
- BAUM C.G. ET AL: "Cellular control of IgE induction by a polyphenol-rich compound. Preferential activation of Th2 cells" J IMMUNOL, vol. 145, no. 3, 1 August 1990 (1990-08-01), page 779-84,

## Beschreibung

Für die Steuerung der Immunantwort in Richtung einer spezifischen humoralen Reaktion, unspezifischen zellulären Reaktion (durch natürliche Killerzellen), spezifischen zellulären Immunantwort (T-Killerzellen mediiert durch T-Helferzellen) oder Immunsuppression sind spezifische Subpopulationen an Lymphozyten verantwortlich. Eine steuernde Rolle spielen dabei die Monozyten.

In Journal of Surgical Research, 89, 169-175 (2000) wird ein Effekt von Curcumin auf die Proliferation von CD4+ und B-Zellen beschrieben.

Es war das Ziel der Untersuchungen eine Möglichkeit zu finden, spezifisch die Proliferation verschiedener Lymphozytensubpopulationen zu fördern. Erfindungsgemäß werden hierzu organische Naturstoffe, die nicht oder kaum in Wasser (wohl aber in anderen Lösungsmitteln) löslich sind auf einen mikropartikulären Carrier aufgebracht, der von Monozyten aufgenommen werden kann.

In Kombination mit einem unspezifischen Immunmodulator wurden die Naturstoffe auf mikropartikulärem Carrier verfüttert. Es zeigte sich dabei, dass bestimmte Stoffe in der Lage sind, spezifisch Subpopulationen and Lymphozyten zu fördern. Die Erfindung ist definiert in den Ansprüchen.

Während der Immunmodulator im m,ukosa-assoziierten lymphatischen Gewebe auf die Lymphozyten einwirkt (Peyersche Plaque oder auch penkapilläre und periendotheliale Lymphozyten) wird der mikropartikuläre Carrier von Monozyten aufgenommen. Die auf den Carrier aufgebrachten Wirkstoffe steuern nunmehr spezifisch die Proliferation bestimmter Lymphozytensubpopulationen. Dies kann beispielsweise geschehen über die Bildung von Interleukin 1 (IL1), Interleukin 10 (IL10), Interleukin 12 (IL12), Tumor-Necrosis-Factor-alpha (T'NF-α), Transforming-Growth-Factor-Alpha (TGF-α) und den Makrophagen-Kolonie-Stimuliernden Faktor (M-CSF).

Die Monozyten können sich über das Blutgefäßsystem im ganzen Körper verbreiten.

### Beispiel:

130 g hochdisperse Kieselsäure werden mit einer Lösung von 8 g Curcumin in 600 ml Ethanol gelöst und anschließend bei 40°C getrocknet.

Diese Zubereitung wird mit 1 kg niedermolekularem Eisen-Alginat als Immunmodulator vermischt.

Die gesamte Mischung wird nunmehr in 18,9 kg pulverisiertem Ascophyllum nodosum eingemischt.

Von der oben genannten Endmischung wurden 500 g einer Tonne Fertigfutter für Ferkel zugemischt.

### Material und Methoden:

30 Ferkel DL x PI aus einer mit PCVII (porcine circo virus II) infizierten Herde waren am Tag 21 abgesetzt worden.

Einstallung an Tag 21, 1 Woche vor dem Absetzen war ein Prästarter gefüttert worden, nach der Einstallung wurde Fertigfutter verfüttert.

Wiegen an Tag 21 und 60 (alle Tiere wurden auf 28-30 kg gefüttert).

Blutentnahme an Tag 49 (vier Wochen nach Einstallung Bestimmung von CD4+; CD8+ and B-cells (CD 22).

| | | |
|---|---|---|
| Zwei Gruppen: | Kontrolle: | 15 Tiere (8 männlich; 7 weiblich) |
| | Versuch: | 15 Tiere (68 männlich, 7 weiblich), 1 Tier wurde während der Einstallung erdrückt |
| | | 500 g Präparat pro t Futter |

### Ergebnis:

| Kontrolle | | | | | |
|---|---|---|---|---|---|
| **Nummer** | **Einstallgewicht kg** | **Gewicht an Tag 60 kg** | **CD4+/µl** | **B-Zellen/µl** | **CD8+µl** |
| **1** | 6,2 | 17,3 | 300 | 104 | 642 |
| **2** | 6,8 | 22,4 | 211 | 78 | 910 |
| **3** | 6,0 | 23,6 | 182 | 92 | 870 |
| **4** | 5,9 | 24,0 | 304 | 64 | 412 |
| **5** | 6,9 | 17,2 | 298 | 52 | 682 |
| **6** | 6,2 | 19,5 | 204 | 64 | 504 |
| **7** | 7,0 | 18,9 | 278 | 90 | 580 |
| **8** | 6,4 | 20,6 | 190 | 60 | 780 |
| **9** | 6,4 | 20,1 | 202 | 102 | 974 |
| **10** | 6,6 | 20,0 | 272 | 112 | 1004 |
| **11** | 6,5 | 20,0 | 185 | 94 | 814 |
| **12** | 6,3 | 17,8 | 212 | 68 | 908 |
| **Mittelwert Standardabweichung** | **6,43 +1-0,34** | **20,12 +/-2,26** | | | |
| **Median (Maximum/Minimum)** | | | **211,5 (182-314)** | **84 (52-112)** | **797 (412-1004)** |

| Versuch | | | | | |
|---|---|---|---|---|---|
| **Nummer** | **Einstallgewicht kg** | **Gewicht an Tag 60 kg** | **CD4+/µl** | **B-Zellen/µl** | **CDB+/µl** |
| **1** | 6,5 | 30,4 | 980 | 430 | 517 |
| **2** | 6,3 | 27,3 | 1040 | 208 | 402 |
| **3** | 6,7 | 26,4 | 1020 | 548 | 517 |
| **4** | 5,9 | 28,3 | 742 | 482 | 836 |
| **5** | 6,8 | 27,9 | 580 | 196 | 572 |
| **6** | 6,6 | 28,4 | 212 | 122 | 394 |
| **7** | 6,0 | 30,0 | 754 | 522 | 647 |
| **8** | 6,8 | 27,3 | 900 | 412 | 474 |
| **9** | 6,8 | 28,1 | 862 | 518 | 852 |
| **10** | 6,4 | 28,4 | 872 | 238 | 642 |
| **11** | 6,4 | 26,9 | 640 | 558 | 709 |
| **12** | 6,7 | 28,4 | 610 | 304 | 974 |
| **13** | 6,5 | 27,9 | 890 | 470 | 900 |
| **Mittelwert Standardabweichung** | **6,49 +1-0,29** | **28,13 +/-1,11** | | | |
| **Median (Maximum/Minimum)** | | | **862 (212-1040)** | **430 (122-558)** | **642 (196-558)** |
| **Median ohne Nr.6** | | | **867** | **450** | **644,5** |

| | | | | | |
|---|---|---|---|---|---|
| 1 Tier tot, ein Spanferkel eliminiert 1 Tier bei Einstallung gestorben, ein Tier gestorben, Nr. 6 offenbar ein Non-Responder. | | | | | |

### Statistik:

| | |
|---|---|
| Gewichte: | Mittelwert und Standardabweichung |
| Zellzahlen: | Rangordnungstest (keine Normalverteilung) ohne Nr. 6 der Versuchsgruppe CD4+: Keine Überlappung |
| | B-Zellen: Keine Überlappung |
| | CD8+: Keine signifikanten Unterschiede auf den Niveaus 0.05 and 0.01 |

### Diskussion:

Bei der vorliegenden Krankheit (PMWS) -einer Immundefizienz- sind charakteristischer Weise CD4+ und B-Zellen stark vermindert. Eine Folge dieses Mangels ist ein stark vermindertes Wachstum. Beide Subpopulationen haben ein beinahe normales Niveau erreicht, wenn die Tiere gleichzeitig das erfindungsgemäße Produkt im Futter verabreicht erhielten. Bei den CD8+-Zellen zeigte sich keine Veränderung,

### Literatur,

Allan, G. M., Kennedy, S., McNeilly, F., Foster, C., Ellis, J. A., Krakowa, S. J., Meehan, B. M. & Adair, B. M.(1999): Experimental reproduction of wasting disease and death by co-infection of pigs with porcine type 2 and porcine parvovirus. Journal of Comparative Pathology 121, 1-11
Allan, G. M., McNeilly, F., Ellis, J., Krakowa, S., Meehan, B., McNair, I., Walker, I. & Kennedy, S. (2000). Experimental infection of CD piglets with PCV2 and PRRS potentiates PCV2 replication. Archives of Virology 145, 2421-2429
Brodersen, B. W., Osorio, F. A., Galeota, J. A., Doster, A. R., Hesse, R. A.(2001): Investigation into the pathogenesis of PMWS, NPPC 98, 225
Krakowa, S., Ellis, J. A., McNeilly, F., Ringler, S., Rings, D. M. & Allan, G. M. (2001): Activation of the immune system is the pivotal event in the production of wasting disease in pigs infected with PCV2. Veterinary Pathology 38, 31-42
Krakowa. S., Ellis, J. A., Meehan, B., Kennedy, S., McNeilly, F. & Allan, G. M. (2000): Viral wasting syndrome of swine: Experimental reproduction of PMWS in gnotobiotic swine by co-infection with PCV2 and PPV. Veterinary Pathology 37, 254-263
Madec, F., Albina, E., Cariolet, R., Hamon, L, Mahe, D., Truong, C., Jesten, A. & Amenna, N. (2000): PMWS in pig: a new challenge for veterinary research and practise. The Pig Journal 45, 69-75
Sarli, G., Mandrioli, L, Laurenti, M., Sidoli, L., Cerati, C., Rolla, G. & Marcato, P. S. (2001): Immunohistochemical characterisation of the lymph node reaction in pig post-weaning multisystemic wasting syndrome (PMWS). Veterinary Immunology and Immunopathology 83 (1-2), 53-67 Sordon, S. D. (2001): Pathogenesis of type II porcine circovirus infection. NPPC 98, 223
Walker, I. W., Konoby, C. A., Jewhurst, V. A., McNair, I., McNeilly, F., Meehan, B. M., Cottrell, T. S., Ellis, J. A. & Allan, G. M. (2000): Development and application of a competitive enzyme-linked immunosorbent assay for the detection of serum antibodies to porcine circovirus type 2. Journal of Veterinary Diagnostic Investigations 12, 400-405

## Patentansprüche

1. Stoff zur spezifischen Proliferationsförderung von als CD4+ ausgebildeten Lymphozytensubpopulationen bei Mensch und Heimtier durch einen Nahrungszusatzstoff, **dadurch gekennzeichnet, dass** Alginsäure und/oder ein Derivat der Alginsäure oder deren Salze mit einem im wesentlichen wasserunlöslichen Naturstoff und einem Carrier kombiniert wird, wobei der Naturstoff Curcumin ist und wobei der Carrier hochdisperse Kieselsäure ist.

2. Stoff nach Anspruch 1, **dadurch gekennzeichnet, dass** der Carrier für den Naturstoff hochdisperse Kieselsäure mit 10-20 nm ist.

3. Verwendung eines Stoffes nach einem der Ansprüche 1-2 zur Herstellung eines Mittels zu spezifischen Proliferationsförderung von als CD4+ ausgebildeten Lymphozytensubpopulationen bei Mensch und Heimtier.

## Claims

1. Material for the specific induction of proliferation of CD4+ lymphocyte subpopulations in humans and domestic animals by means of a food additive, **characterized in that** alginic acid and/or a derivative of alginic acid or salts thereof are combined with an essentially water-insoluble natural substance and a carrier, with the natural substance being curcumin and the carrier being colloidal silica.

2. Material according to Claim 1, **characterized in that** the carrier for the natural substance is colloidal silica at 10-20 nm.

3. Use of a material according to either of Claims 1 and 2 for preparing a means for the specific induction of proliferation of CD4+ lymphocyte subpopulations in humans and domestic animals.

## Revendications

1. Substance pour la stimulation spécifique, par un additif alimentaire, de la prolifération de sous-populations lymphocytaires différenciées en CD4+ chez l'homme et les animaux domestiques, **caractérisée en ce que** de l'acide alginique et/ou un dérivé de l'acide alginique, ou des sels de ce dernier, sont combinés à une substance naturelle pour l'essentiel non hydrosoluble et à un support, la substance naturelle étant la curcumine, le support étant une silice hautement dispersée.

2. Substance selon la revendication 1, **caractérisée en ce que** le support de la substance naturelle est une silice hautement dispersée de 10-20 nm.

3. Utilisation d'une substance selon l'une des revendications 1-2 pour préparer un produit destiné à la stimulation spécifique de la prolifération de sous-populations lymphocytaires différenciées en CD4+ chez l'homme et les animaux domestiques.
